# FASCICULE DE BREVET EUROPEEN

(11) **EP 3 980 087 B1**
(45) Date de publication et mention de la délivrance du brevet: **11.10.2023**
(21) Numéro de dépôt: 20729774.8
(22) Date de dépôt: 04.06.2020
(51) Int. Cl.: A61L 27/36, A61L 27/54, A61L 27/52

(54) **LENTILLE BIOLOGIQUE COMPRENANT UNE MEMBRANE AMNIOTIQUE**
BIOLOGISCHE LINSE MIT EINER AMNIOTISCHEN MEMBRAN
BIOLOGICAL LENS COMPRISING AN AMNIOTIC MEMBRANE

(30) Priorité: 04.06.2019 FR 1905931; 18.10.2019 FR 1911673
(43) Date de publication de la demande: 13.04.2022
(73) Titulaire: TBF Genie Tissulaire (TBF), 69780 Mions (FR)
(72) Inventeur: BARNOUIN, Laurence, 38460 Vénérieu (FR)
(74) Mandataire: Tripoz, Inès
(86) Numéro de dépôt international: PCT/EP2020/065565
(87) Numéro de publication internationale: WO 2020/245324

(56) Documents cités:
- US-A1- 2008 181 967
- US-A1- 2009 143 792

## Description

La présente invention concerne le domaine des lentilles biologiques. De telles lentilles appelées également « lentilles pansement » comprennent une membrane amniotique et sont utilisées notamment pour des applications médicales, par exemple dans le traitement des surfaces oculaires. Par concision, on utilisera uniquement la terminologie « lentille » ci-après.

### Art antérieur

La membrane chorioamniotique, qui sépare le foetus de l'endomètre de la mère chez les mammifères, inclut la membrane amniotique, ou amnios, et la membrane choriale, ou chorion. Ces deux membranes sont reliées par une membrane tissulaire spongieuse, aussi appelée couche spongieuse, constituée entre autres de collagène et de protéoglycanes, la membrane tissulaire spongieuse comportant des ponts protéiques, attachés de part et d'autre à l'amnios d'une part, et au chorion, d'autre part.

La membrane amniotique est la couche la plus interne de la membrane chorioamniotique. Elle a pour rôle de protéger le foetus et de maintenir autour de lui le liquide amniotique. Cette membrane amniotique est un tissu très fin, transparent qui comporte plusieurs couches, à savoir une couche épithéliale, une membrane basale, une couche compacte et une couche fibroblastique.

Non vascularisée et non innervée, la membrane amniotique est riche en collagène et en divers facteurs de croissance, et présente des propriétés participant au processus de cicatrisation.

La membrane amniotique, obtenue à partir du placenta après un accouchement, est un tissu utilisé depuis plus de cent ans dans le traitement des brûlures et des blessures. En effet, dès 1910, Davies utilisait des membranes foetales tant sur des brûlures que sur des tissus ulcérés. Trelford et Trelford-Sauder rapportent qu'en 1935 des auteurs ont publié des applications cliniques de la membrane amniotique en vaginoplastie, reconstruction conjonctivale, traitement des brûlures ou des blessures, et traitements relatifs à l'adhérence intra-abdominale. Trelford *et al.* rapportent aussi qu'en 1952 Douglas a utilisé de l'amnios pour traiter des blessures étendues. Pour la première fois, il fut indiqué que la couche stromale de la membrane amniotique, comprenant la couche compacte et la couche fibroblastique, permettait une adhérence plus importante de la greffe, et donc de son efficacité. En 1972, les travaux de Trelford *et al.* ont confirmé ce fait. Gindraux *et al.* rapportent qu'à partir de 1972, et surtout depuis sa redécouverte en 1995, d'autres auteurs ont confirmé toutes les applications cliniques présentées précédemment, et ont également signalé de nouvelles indications telles que le tractus génito-urinaire, l'estomac, le larynx, la cavité orale, la tête et le cou, que ce soit dans des essais cliniques ou des rapports de cas.

Dans le domaine de l'ophtalmologie, c'est en 1940 que De Roetth a utilisé pour la première fois une membrane amniotique en ophtalmologie reconstructive pour traiter des altérations conjonctivales de patients.

Ce type de produit biologique est particulièrement apprécié dans le traitement des maladies inflammatoires de la cornée, telles que les ulcères cornéens ; ou dans le traitement de destructions de la surface de la cornée, induites par exemples par des brûlures à l'acide, le syndrome de Stevens-Johnson ou le syndrome de Lyell.

Dans ce type de pathologies, il existe donc une destruction progressive de l'épithélium cornéen. Cette destruction s'accompagne souvent par un remplacement par l'organisme d'un épithélium anormal, entraînant une vascularisation et une inflammation locale qui conduisent à une perte de la transparence du tissu cornéen.

Ces pathologies ne concernant que l'épithélium cornéen (la surface de la cornée), le remplacement de la cornée est inadapté. L'utilisation de membrane amniotique en tant que greffe est connue pour permettre la cicatrisation de la cornée du patient, en évitant ainsi le remplacement de celle-ci.

En effet, la greffe de membrane amniotique permet au niveau local la fourniture d'une structure, avasculaire et acellulaire, servant de membrane basale de support à l'épithélium cornéen. En conséquence, cette technique évite les phénomènes de rejet et d'inflammation locale, tout en facilitant la cicatrisation épithéliale cornéenne.

Outre la greffe, la membrane amniotique peut également être utilisée comme pansement, ou patch, lorsque sa propre surface épithéliale est suturée de sorte à être en contact avec la surface de l'œil. Dans ce cas, la membrane délivre les facteurs biologiques dont elle est riche vers la cornée du patient afin de promouvoir la cicatrisation de celle-ci.

Les membranes amniotiques sont particulièrement riches en facteurs de croissance de type EGF (Epidermal Growth Factor), TGF (Transforming Growth Factor) et KGF (Keratinocyte Growth Factor), ainsi qu'en acide hyaluronique. Ces facteurs de croissance et l'acide hyaluronique sont particulièrement adaptés pour promouvoir les processus biologiques de cicatrisation, notamment la cicatrisation de l'épithélium cornéen.

Toutefois, que ce soit en greffe ou en pansement, ces techniques présentent pour inconvénient de devoir suturer la membrane amniotique à la surface du globe oculaire, ce qui nécessite un geste chirurgical et une anesthésie du patient.

Il existe donc un besoin pour un appareil médical permettant l'utilisation d'une membrane amniotique dans le traitement des lésions de l'épithélium cornéen, sans qu'il soit nécessaire de procéder à la suture de la membrane pour la maintenir en place.

Plusieurs supports pour membranes amniotiques, destinés à être utilisés dans le traitement des lésions de l'épithélium cornéen, sans qu'il soit nécessaire de procéder à la suture desdits supports, ont été proposés.

Dans le document WO2003077794 au nom de TISSUETECH sont décrits différents dispositifs où une membrane amniotique est fixée sur un anneau support de différentes manières. La membrane peut être fixée sur l'anneau support à l'aide d'une colle, s'enrouler autour de l'anneau support, être insérée dans l'anneau support, être clipsée à l'anneau support à l'aide d'un second anneau.

Dans le document US2009/143792 est décrit un dispositif où une membrane est fixée entre deux anneaux disposés de manière concentriques l'un par rapport à l'autre. L'anneau interne est de structure rigide, l'anneau externe est de structure élastique. Les deux anneaux associés présentent une surface interne commune en forme de cône tronquée de sorte à s'adapter à la convexité de l'œil. La membrane amniotique étant clipsée entre 2 anneaux, une contrainte mécanique est exercée sur celle-ci pouvant conduire à des risques de rupture au montage et à l'usage.

La demande WO2016138025 au nom de TISSUETECH divulgue un dispositif de lentille comprenant un support de membrane sous forme de structure tubulaire flexible pouvant être en différents matériaux incluant des tissus biologiques. Ce dispositif requiert l'utilisation de moyens flexibles pour fixer la membrane sur le support.

Le problème posé par ces dispositifs est qu'ils ont tous recourt à des moyens de fixation de la membrane amniotique à leur support. Lors de cette étape de fixation, mais également à l'usage, des contraintes mécaniques sont appliquées à la membrane amniotique qui est un matériau fragile, pouvant ainsi entraîner une rupture ou une dégradation de la membrane. La manipulation lors de l'assemblage du dispositif et son utilisation se doit donc d'être très minutieuse et peut s'avérer contraignante.

Le dispositif selon la présente invention permet de résoudre le problème posé par les dispositifs de lentilles de l'art antérieur.

Le dispositif selon la présente invention ne requiert pas de moyens de fixation de la membrane au support, en ce qu'il s'agit du support qui est appliqué sur la membrane sous forme de 2 couches de gel formant une structure annulaire entourant le contour extérieur de la membrane, chacune des 2 couches étant appliquée sur une face différente du contour externe de la membrane. Une étape ultérieure de solidification du gel permettra le maintien de la membrane entre les 2 couches de gel formant un support rigide pour la membrane. De ce fait aucune contrainte mécanique n'est appliquée à la membrane lors de l'assemblage du dispositif, ni lors de son utilisation.

### Résumé de l'invention

La présente invention concerne un dispositif, utilisable comme lentille 10, comprenant :
- une membrane amniotique 20 ;
- une structure annulaire 30 solidaire de ladite membrane et disposée à la périphérie de celle-ci, caractérisé en ce que ladite structure annulaire 30 est sous forme d'un matériau durcissable, ledit matériau étant un gel.

Dans un mode de réalisation la membrane amniotique 20, est de forme sensiblement circulaire.

Dans un mode de réalisation la membrane amniotique 20, est de forme sensiblement ovale ou sous forme d'ellipse.

Dans un mode de réalisation la membrane amniotique 20, est en forme de calotte sensiblement circulaire.

Dans un mode de réalisation la membrane amniotique 20, est en forme de calotte sensiblement ovale ou elliptique.

La présente invention concerne également un procédé de fabrication d'un dispositif utilisable comme lentille 10 comprenant les étapes de :
a) on dispose d'un support 50 ;
b) on prépare une première couche de gel 30 de forme annulaire ;
c) on dispose une membrane amniotique 20 sur ladite première couche 30 de façon à mettre en contact la périphérie de ladite membrane et le gel ;
d) on prépare une seconde couche de qel 30 de forme annulaire celle-ci étant appliquée sur la membrane et la première couche de façon à ce que la périphérie de la membrane soit entourée de gel ;
e) on procède à une étape de solidification du gel et on obtient un dispositif utilisable comme lentille 10 biologique.

La présente invention concerne également un support permettant l'assemblage d'un dispositif utilisable comme lentille 10 caractérisé en ce qu'il comprend :
- un dôme à base sensiblement circulaire ou elliptique 51 ;
- un socle 52, comprenant une cavité, ou gorge 53, de forme convexe à la périphérie du dôme.

### Description des figures

La FIGURE 1 illustre une coupe transversale d'un mode de réalisation d'un support selon l'invention,
La FIGURE 2 illustre une coupe transversale d'un autre mode de réalisation d'un support selon l'invention, dans lequel le dôme est amovible,
La FIGURE 3 illustre une vue de dessus d'un mode de réalisation d'un support selon l'invention,
La FIGURE 4 illustre une coupe transversale d'un mode de réalisation d'un support selon l'invention, sur lequel une membrane est positionnée, la périphérie de la membrane étant prise dans la masse d'une structure annulaire, La FIGURE 5 illustre le mode de réalisation dans lequel la périphérie de la membrane est en outre prise dans la masse d'une structure annulaire stabilisée par un anneau.
La FIGURE 6 illustre une variante de la FIGURE 5, dans lequel l'anneau de stabilisation présente une autre forme,
La FIGURE 7 illustre une vue de dessus d'un mode de réalisation d'une lentille selon l'invention,
La FIGURE 8 illustre une vue de dessus d'un autre mode de réalisation d'une lentille selon l'invention, comprenant un anneau de stabilisation pris dans la masse d'une structure annulaire,
La FIGURE 9 illustre une vue de dessus d'un autre mode de réalisation d'une lentille selon l'invention, présentant une forme globalement ovale ou elliptique. La FIGURE 10 illustre l'évolution de l'activité totale de gentamycine relarguée d'un dispositif de lentille selon l'invention rempli de gelée de Wharton imprégnée de ladite gentamycine en UI/ml (axe des ordonnées) en fonction du temps en heures (axe des abscisses).

### Description détaillée de l'invention

La présente invention concerne un dispositif de lentille 10 biologique ou lentille 10 pansement à des fins d'applications médicales, par exemple dans le traitement des surfaces oculaires.

Par convention, on fera référence à un dispositif selon l'invention positionné sur l'œil d'un individu étant un être humain ou un mammifère en général allongé dans un plan horizontal et dont l'axe optique est vertical. Dans le cas d'une application vétérinaire, le dispositif sera adapté notamment en fonction de la taille et la forme de l'œil de l'animal.

La présente invention concerne un dispositif, utilisable comme lentille 10, comprenant :
- une membrane amniotique 20 ;
- une structure annulaire 30 solidaire de ladite membrane et disposée à la périphérie de celle-ci, caractérisé en ce que ladite structure annulaire 30
est sous forme d'un matériau durcissable, ledit matériau étant un gel.

Dans un mode de réalisation la membrane amniotique 20, est de forme sensiblement circulaire.

Dans un mode de réalisation la membrane amniotique 20, est de forme sensiblement ovale ou sous forme d'ellipse.

Dans un mode de réalisation la membrane amniotique 20, est en forme de calotte sensiblement circulaire.

Dans un mode de réalisation la membrane amniotique 20, est en forme de calotte sensiblement ovale ou elliptique.

### Membrane amniotique

Par « membrane amniotique », on entend l'enveloppe tissulaire qui se développe autour de l'embryon, puis du foetus, chez le mammifère durant la grossesse. Elle a pour rôle de protéger l'organisme en développement en maintenant autour de lui le liquide amniotique. Elle s'accole à la deuxième membrane qui est le chorion. La membrane amniotique inclut les sous-couches physiologiques suivantes : la couche cellulaire épithéliale, la membrane basale, la couche compacte, la couche fibroblastique, la couche spongieuse.

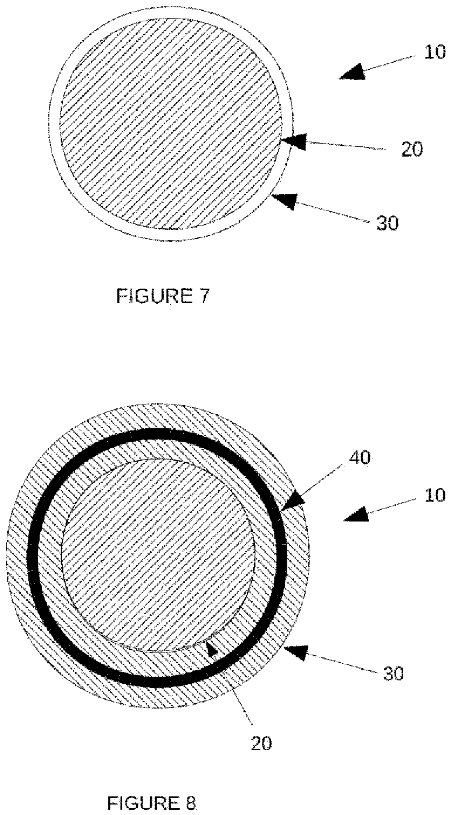

La membrane amniotique 20 est souple. Cette propriété est avantageusement utilisée par la présente invention. En effet, selon l'invention une membrane amniotique 20 est prédécoupée selon une forme prédéfinie, par exemple sensiblement circulaire, comme illustrée sur la figure 8, ovale ou elliptique. Grâce à sa flexibilité intrinsèque, la membrane amniotique 20 peut présenter une forme convexe une fois le dispositif selon l'invention positionné sur une cornée, c'est à dire que la membrane 20 épouse la forme de la cornée.

La surface équivalente de la prédécoupe de la membrane amniotique 20 dépend du diamètre de la structure annulaire. Cette surface est inférieure ou égale à la surface équivalente de l'œil.

Dans un mode de réalisation, le dispositif selon l'invention est caractérisé en ce que la membrane amniotique 20 est stérile.

Les membranes amniotiques étant des tissus biologiques, le risque de transmission d'une infection d'un donneur vers un receveur, ou de contamination lors de la collecte du tissu à l'accouchement, lors des traitements préparatoires du tissu, ou lors du transport de ce tissu est important. De fait, un procédé efficace tant de désinfection que de conservation de ces tissus est vital pour la sécurité des patients.

Dans un mode de réalisation, le dispositif selon l'invention est caractérisé en ce que la membrane amniotique 20 est viro-inactivée.

Par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un tel procédé est par exemple décrit dans le document WO2017/140914 au nom de TBF GENIE TISSULAIRE.

Dans un mode de réalisation, le dispositif selon l'invention est caractérisé en ce que la membrane amniotique 20 est viro-inactivée selon les deux étapes de viro-inactivation chimiques du procédé décrit dans WO2017/140914.

Dans un mode de réalisation, le dispositif selon l'invention est caractérisé en ce que la membrane amniotique 20 est obtenue selon le procédé décrit dans WO2017/140914.

Dans un mode de réalisation, le dispositif selon l'invention est caractérisé en ce que la membrane amniotique 20 est constituée de deux fragments de membrane amniotique superposés de façon à ce que les faces épithéliales de chacun des fragments soient sur les faces externes de la membrane double épaisseur réalisée par la superposition.

La membrane amniotique 20 en double épaisseur est réalisée de façon à ce que pour chaque épaisseur de membrane amniotique l'épithélium soit orienté de façon à être en contact avec l'œil pour une épaisseur, et en contact avec l'intérieur des paupières pour l'autre épaisseur. Une telle configuration de membrane amniotique 20 est particulièrement utile dans le traitement du syndrome de Stevens-Johnson ou du syndrome de Lyell.

### Structure annulaire

Le dispositif selon l'invention comprend une structure annulaire 30, solidaire de la membrane 20 et disposée à la périphérie de celle-ci.

En utilisation comme lentille 10, la membrane est au contact de la cornée. Elle est maintenue en forme grâce à la structure annulaire 30.

Par « structure annulaire », on entend une structure biocompatible de forme arrondie, par exemple en forme de cercle ou en forme d'ovale ou d'ellipse. Cette structure est dite solidaire de la membrane 20 car elle ne peut en aucun cas s'en dissocier. Une fois mise en place, la structure annulaire 30 ne peut être dissociée de la membrane 20 sans que l'intégralité de ladite structure annulaire 30 ne soit détruite. La membrane est en effet complètement entourée sur toutes ses faces et ses bords dans la structure annulaire qui est formée autour de la membrane.

Lorsque la structure annulaire est ciculaire elle est définie par son diamètre lorsqu'elle est de forme elliptique ou ovale elle sera définie par un diamètre équivalent.

Le diamètre est fixé par la taille de la surface oculaire à traiter, cette surface étant inférieure ou égale à la surface équivalente de l'oeil.

Dans un mode de réalisation, le dispositif selon l'invention est caractérisé en ce que la structure annulaire 30 possède un diamètre compris entre 1 et 3 cm.

Dans un mode de réalisation, le dispositif selon l'invention est caractérisé en ce que la structure annulaire 30 possède un diamètre compris entre 2 et 2,5 cm.

La membrane amniotique 20 présente donc un diamètre équivalent. Par « diamètre équivalent », on entend l'une des définitions ci-dessous, dépendant de la forme de la membrane amniotique 20.
- lorsque la membrane amniotique 20 se présente sous forme de disque, le diamètre équivalent est le diamètre du cercle représentant la circonférence du disque;
- lorsque la membrane amniotique 20 se présente sous forme d'ellipse, le diamètre équivalent est compris entre le double de la longueur du petit axe, également appelé petit rayon et le double de la longueur du grand axe, également appelé grand rayon, de l'ellipse ;
- lorsque la membrane amniotique 20 se présente sous forme d'ovale, c'est à dire une courbe plane fermée convexe,
   * le diamètre équivalent est inférieur ou égal à la longueur de l'axe de symétrie lorsque la courbe plane fermée convexe possède un unique axe de symétrie ; et
   * le diamètre équivalent est compris entre la longueur de l'axe de symétrie le plus long et la longueur de l'axe de symétrie le plus court, lorsque la courbe plane fermée convexe possède deux axes de symétrie orthogonaux."

On prévoit de préférence que le diamètre équivalent de la prédécoupe de la membrane amniotique 20 est inférieur ou égal au diamètre moyen d'un oeil humain adulte. En l'espèce, le diamètre équivalent de la prédécoupe de la membrane amniotique 20 est compris entre 2 cm et 2,5 cm.

Dans un mode de réalisation, le dispositif selon l'invention est caractérisé en ce que la structure annulaire 30 possède une épaisseur comprise entre 1 et 4 mm

La structure annulaire 30 présente de préférence une symétrie, en l'espèce une symétrie de révolution sur l'exemple illustré la figure 7 ou la figure 8. Avantageusement lorsque le dispositif selon l'invention est positionné sur un oeil, l'axe Z-Z de symétrie de révolution est confondu avec l'axe optique de l'œil.

La structure annulaire 30 est constituée d'un matériau durcissable, c'est à dire que le matériau constitutif présente une consistance de fluide (de préférence Newtonien), de pâte ou de gel lors de la fabrication du dispositif selon l'invention, et une consistance solide lors de l'utilisation du dispositif selon l'invention. Par consistance solide on entend que le matériau ne se déforme pas lorsqu'on le manipule.

Par matériau constitutif, on entend un unique matériau, une combinaison ou un assemblage de matériaux constitutifs durcissables.

La solidification peut se faire par action chimique ou physique, par exemple par lyophilisation.

Par « lyophilisation », on entend une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.

Le matériau constitutif est un gel durcissable.

Par « gel » on entend une structure associée lâche, immergée dans une phase fluide, ou une masse de nature colloïdale homogène, de consistance molle.

Dans un mode de réalisation, le dispositif selon l'invention est caractérisé en ce que la structure annulaire 30 est sous forme de gel durcissable choisi parmi le groupe des gels biocompatibles de polysaccharides, des gels de polymères de galactose, des gels de tissus biologiques, pris seuls ou en combinaison.

Dans un mode de réalisation, le dispositif selon l'invention est caractérisé en ce que la structure annulaire 30 est stérile, c'est à dire exempte de germe, que ce soit à l'état naturel ou après stérilisation.

Dans un mode de réalisation, le dispositif selon l'invention est caractérisé en ce que la structure annulaire 30 est en gelée de Wharton.

### Gelée de Wharton

La gelée de Wharton est un tissu conjonctif gélatineux entourant la veine et les deux artères du cordon ombilical des mammifères. La gelée de Wharton est une substance particulièrement riche en éléments constitutifs de la matrice extracellulaire des tissus conjonctifs, notamment en glycosaminoglycanes et protéoglycanes ; ainsi qu'en fibres de collagène (types I, III, IV et V). La gelée de Wharton comprend également de nombreux facteurs de croissance tels que, notamment, les facteurs de croissance des fibroblastes (FGF), les facteurs de croissance I ressemblant à l'insuline (IGF-I), les facteurs de croissance transformant (TGF), les facteurs de croissance dérivés des plaquettes (PDGF) et les facteurs de croissance épidermique (EGF).

Ces propriétés structurelles de la gelée de Wharton peuvent être utilisées pour leurs propriétés d'aide à la cicatrisation des lésions, notamment des lésions cutanées ou des lésions de la surface oculaire. En effet, ces éléments constitutifs de la gelée de Wharton participent à l'amélioration des processus biologiques de cicatrisation et de réduction de l'inflammation chez un patient.

Par « gelée de Wharton », on entend le tissu biologique gélatineux présent dans le cordon ombilical des mammifères dont la veine et les deux artères naturellement incluses au sein dudit tissu biologique gélatineux ont été retirées. Dans la présente invention, le terme « gelée de Wharton » peut être entendu comme comprenant ou non la membrane amniotique entourant la gelée de Wharton du cordon ombilical. Dans la présente invention, le terme « gelée de Wharton » est entendu comme ne comprenant pas de fibres de collagène épaisses et/ou de lacunes et parois vasculaires (villosités et chambres intervilleuses).

La demande WO2019/038411 au nom de TBF GENIE TISSULAIRE divulgue un biomatériau constituant en un broyat de gelée de Wharton et son procédé de viro-inactivation.

Dans un mode de réalisation, le dispositif selon l'invention est caractérisé en ce que la structure annulaire 30 en gelée de Wharton est viro-inactivée.

Dans un mode de réalisation, le dispositif selon l'invention est caractérisé en ce que la structure annulaire 30 en gelée de Wharton est viro-inactivée selon les deux étapes de viro-inactivation chimiques du procédé décrit dans WO2019/038411.

Dans un mode de réalisation, le dispositif selon l'invention est caractérisé en ce que la structure annulaire 30 en gelée de Wharton est obtenue selon le procédé décrit dans WO2019/038411.

Dans un mode de réalisation, le dispositif selon l'invention est caractérisé en ce que la structure annulaire 30 en gelée de Wharton comprend au moins un principe actif.

Le au moins un principe actif peut être un principe actif ajouté, qui n'est pas naturellement présent dans la structure annulaire 30 en gelée de Wharton native.

Dans un mode de réalisation, le dispositif selon l'invention est caractérisé en ce que la structure annulaire 30 en gelée de Wharton comprend au moins un principe actif choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des antifongiques, des anti-allergiques, des anesthésiques, ou des protéines immunoadhésives, des agents permettant d'éviter les sécheresses oculaires, seuls ou en combinaisons.

Dans un mode de réalisation, le dispositif selon l'invention est caractérisé en ce que la structure annulaire 30 en gelée de Wharton comprend au moins un principe actif choisi dans le groupe constitué des antibiotiques. Quelques exemples d'antibiotiques utilisables sont donnés ci-après : tétracyclines (daunomycine, tétracycline, chloretetracycline, oxytétracycline, etc.), glycopeptides (vancomycine, etc.), aminoglycosides (gentamycine, etc.), aminosides (tobramycine, néomycine, etc.), fluoroquinolones (ciprofloxacine, moxifloxacin, etc.), quinolones (gatifloxacine, etc.), polypeptides (bacitracine, polymyxine, etc.), phénicolés (chloramphénicol, etc.), macrolides (erythromycine, etc.), sulfonamides (sulfacétamide, sulfaméthoxazole, sulfisoxazole, etc.), céphalosporines (céfradoxil, céfoxitine, etc.), et tout autre antibiotique.

Dans un mode de réalisation, le dispositif selon l'invention est caractérisé en ce que la structure annulaire 30 en gelée de Wharton comprend au moins un principe actif choisi dans le groupe constitué des anti-inflammatoires stéroïdiens (AIS) et/ou non-stéroïdiens (AINS). Quelques exemples d'AIS sont donnés ci-après : triamcinolone, dexaméthasone, prednisolone, hydrocortisone, corticostérone, fluocinolone, prednisolone, méthylprednisolone, fluorométholone, betaméthasone, tétrahydrocortisol, riméxolone, etc. Quelques exemples d'AINS sont donnés ci-après : indométacine, népafénac, diclofénac, bromfénac, kétorolac, suprofène, etc.

Dans un mode de réalisation, le dispositif selon l'invention est caractérisé en ce que la structure annulaire 30 en gelée de Wharton comprend au moins un principe actif choisi dans le groupe constitué des immunosuppresseurs, une liste non-limitative est donnée ci-après : déxaméthasone, bétaméthasone, etc.

Dans un mode de réalisation, le dispositif selon l'invention est caractérisé en ce que la structure annulaire 30 en gelée de Wharton comprend au moins un principe actif choisi dans le groupe constitué des antiviraux, une liste non-limitative est donnée ci-après : ganciclovir, trifluorothymidine, aciclovir, DDI, AZT, foscarnet, vidarabine, trifluorouridine, idoxuridine, ribavirine, inhibiteurs de protéase, agent anti-cytomégalovirus, etc.

Dans un mode de réalisation, le dispositif selon l'invention est caractérisé en ce que la structure annulaire 30 en gelée de Wharton comprend au moins un principe actif choisi dans le groupe constitué des antifongiques, une liste non-limitative est donnée ci-après : fluconazole, nitrofurazone, amphotéricine B, kétoconazole, etc.

Dans un mode de réalisation, le dispositif selon l'invention est caractérisé en ce que la structure annulaire 30 en gelée de Wharton comprend au moins un principe actif choisi dans le groupe constitué des antiallergiques, une liste non-limitative est donnée ci-après : méthapyriline, chlorpheniramine, pyrilamine, prophenpyridamine, etc.

Dans un mode de réalisation, le dispositif selon l'invention est caractérisé en ce que la structure annulaire 30 en gelée de Wharton comprend au moins un principe actif choisi dans le groupe constitué des anesthésiques, une liste non-limitative est donnée ci-après : lidocaïne, mépivacaïne, etc.

Dans un mode de réalisation, le dispositif selon l'invention est caractérisé en ce que la structure annulaire 30 en gelée de Wharton comprend au moins un principe actif choisi dans le groupe constitué des agents permettant d'éviter les sécheresses oculaires, une liste non-limitative est donnée ci-après : azithromycine, ciclosporine, lubrifiants etc...

### Anneau de stabilisation

Dans un mode de réalisation, le dispositif selon l'invention est caractérisé en ce que la structure annulaire 30 comprend en outre un anneau de stabilisation 40.

Par « anneau de stabilisation » on entend un élément annulaire, de matière par exemple polymérique, métallique ou biologique, de consistance rigide ou flexible (élastique), et possédant un diamètre équivalent prédéfini, et autour duquel la membrane 20 est enroulée, ou sur lequel la membrane 20 est apposée. Le diamètre équivalent de l'anneau est lié au diamètre de la structure annulaire. L'anneau de stabilisation possède une épaisseur variable en fonction de la forme de la structure annulaire désirée (ronde, ovale...).

Dans un mode de réalisation, le dispositif selon l'invention est caractérisé en ce que l'anneau de stabilisation 40 est stérile.

L'anneau de stabilisation 40 est par exemple un matériau polymère, un métal ou un alliage, de préférence biocompatible.

Dans un mode de réalisation, le dispositif selon l'invention est caractérisé en ce que l'anneau de stabilisation 40 est en matériau plastique biocompatible.

Dans un mode de réalisation, le dispositif selon l'invention est caractérisé en ce que l'anneau de stabilisation 40 est en polypropylène ou en PVC.

Les matériaux plastiques biocompatibles peuvent également être par exemple du polyéthylène (PE), du polytétrafluoroéthylène (PTFE), du polyétheréthercétone (PEEK), de l'acide polylactique (PLA), etc.

Dans un mode de réalisation, le dispositif selon l'invention est caractérisé en ce que l'anneau de stabilisation 40 est en matière biologique.

Des exemples de matières biologiques utilisables sont du derme, de la paroi de cordon ombilical, ou tout tissu biologique ayant une tenue adéquate pour pouvoir stabiliser la structure annulaire.

Dans un mode de réalisation, le dispositif selon l'invention est caractérisé en ce que l'anneau de stabilisation 40 est un segment de vaisseau ombilical rempli de gelée de Wharton.

L'anneau est constitué par emboîtage bout à bout des extrémités du segment de vaisseau ombilical ledit segment étant rempli de gelée de Wharton puis lyophilisé pour assurer une rigidité à l'ensemble.

On dispose ainsi d'un dispositif avec une structure annulaire stabilisée qui est 100% biologique.

L'anneau de stabilisation 40 présente une face interne, destiné à être au contact de la cornée par l'intermédiaire de la structure annulaire 30.

Dans un mode de réalisation, le dispositif selon l'invention est caractérisé en ce que l'épaisseur de l'anneau de stabilisation 40 est comprise entre 0,1 mm et 3 mm.

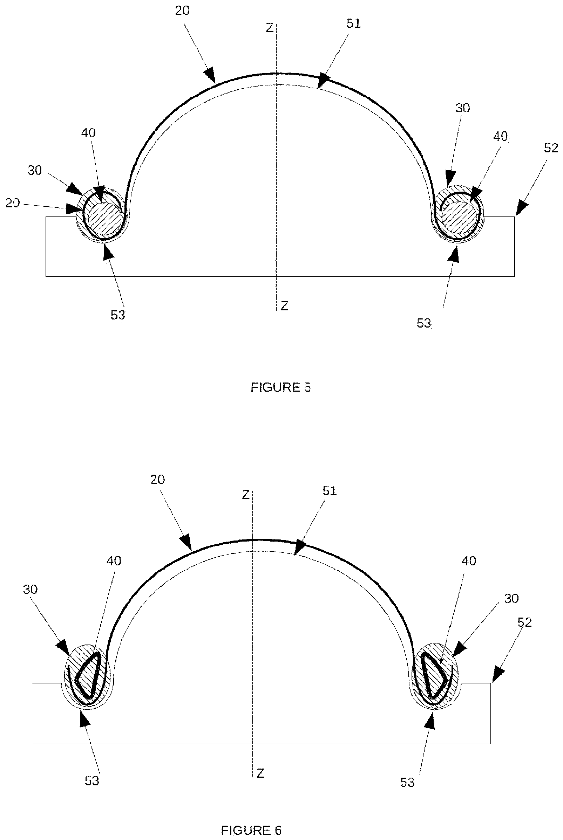

Dans un mode de réalisation, le dispositif selon l'invention est caractérisé en ce que la section transversale d'un anneau de stabilisation 40 présente une face interne de forme convexe, de sorte à épouser la courbure de la cornée d'un oeil, comme illustré sur la figure 6.

La section d'un anneau de stabilisation 40 peut également être ronde ou quelconque, sous réserve qu'elle ne présente pas d'angle aigu.

De préférence, la face interne de l'anneau de stabilisation 40 épouse localement la forme du dôme 51 du support 50 décrit ultérieurement. La forme de la face interne est de préférence arrondie ou tronconique.

### Utilisation

Le dispositif selon l'invention est destiné à être utilisé dans le traitement des lésions oculaires.

Le dispositif selon l'invention est destiné à être utilisé dans le traitement des maladies inflammatoires de la conjonctive et cornée.

Le dispositif selon l'invention est destiné à être utilisé dans le traitement des infections de la conjonctive et cornée.

Le dispositif selon l'invention est destiné à être utilisé dans le traitement de destructions de la surface de la conjonctive et cornée.

Le dispositif selon l'invention est destiné à être utilisé dans le traitement de lésions de la conjonctive et cornée choisies dans le groupe constitué des ulcères cornéens, des brûlures, du syndrome de Stevens-Johnson, du syndrome de Lyell, les keratoconjonctivites immunitaires, allergiques, inflammatoire, traumatiques.

Le dispositif selon l'invention est destiné à être utilisé dans le traitement des sécheresses oculaires.

### Procédé de fabrication

La présente invention concerne également un procédé de fabrication d'un dispositif utilisable comme lentille 10 comprenant les étapes de :
a) on dispose d'un support 50 ;
b) on prépare une première couche de gel 30 de forme annulaire ;
c) on dispose une membrane amniotique 20 sur ladite première couche 30 de façon à mettre en contact la périphérie de ladite membrane et le gel;
d) on prépare une seconde couche de gel 30 de forme annulaire celle-ci étant appliquée sur la membrane et la première couche de façon à ce que la périphérie de la membrane soit entourée de gel ;
e) on procède à une étape de solidification du gel et on obtient un dispositif utilisable comme lentille 10 biologique, comme illustré sur la figure 7.

Dans un mode de réalisation, le procédé selon l'invention est caractérisé en ce qu'il comprend une étape de pose d'un anneau de stabilisation 40 entre l'étape c) et d) comme illustré sur la figure 8.

Dans un mode réalisation, le procédé selon l'invention est caractérisé en ce qu'aux étapes b) et d) le gel 30 est de la gelée de Wharton.

Dans un mode réalisation, le procédé selon l'invention est caractérisé en ce qu'à l'étape e) la solidification de la gelée de Wharton est réalisée par une lyophilisation.

Dans un mode réalisation, le procédé selon l'invention est caractérisé en ce qu'à l'étape e) la solidification par lyophilisation de la gelée de Wharton est réalisée avec ajout de tréhalose.

Dans un mode de réalisation, la présente invention concerne également un procédé de fabrication d'un dispositif utilisable comme lentille 10 comprenant les étapes de :
a) on dispose d'un support 50 ;
b) on prépare une première couche de gel 30 de forme annulaire ;
c) on dispose d'un anneau de stabilisation 40 sur lequel est apposée ou enroulée une membrane amniotique 20, que l'on pose sur ladite première couche de façon à mettre en contact l'anneau et le gel;
d) on prépare une seconde couche de gel 30 de forme annulaire celle-ci étant appliquée sur la membrane et la première couche de façon à ce que la périphérie de la membrane et l'anneau soit entourés de gel ;
e) on procède à une étape de solidification du gel.

Dans un mode réalisation, le procédé de fabrication d'un dispositif utilisable comme lentille 10 selon l'invention est caractérisé en ce qu'aux étapes b) et d) le gel 30 est de la gelée de Wharton.

Dans un mode réalisation, le procédé de fabrication d'un dispositif utilisable comme lentille 10 selon l'invention est caractérisé en ce qu'à l'étape e) la solidification de la gelée de Wharton est réalisée par une lyophilisation.

Dans un mode réalisation, le procédé de fabrication d'un dispositif utilisable comme lentille 10 selon l'invention est caractérisé en ce qu'à l'étape e) la solidification par lyophilisation de la gelée de Wharton est réalisée avec ajout de tréhalose.

### Support

La présente invention concerne également un support 50 permettant l'assemblage d'un dispositif selon l'invention, utilisable comme lentille 10.

Avantageusement, le support 50 selon l'invention agit comme un demi-moule permettant de pré contraindre la membrane amniotique 20, et de solidariser ladite membrane amniotique 20 et la structure annulaire 30.

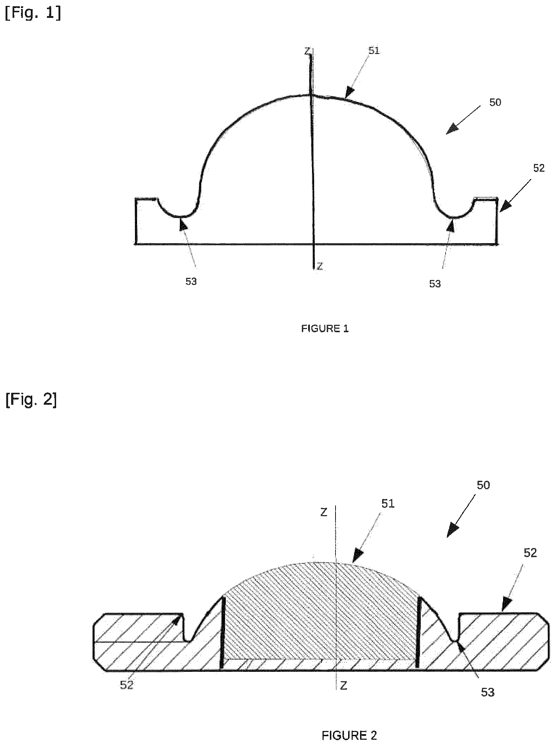

Le support 50 selon l'invention, dont un exemple est illustré sur la figure 1, est essentiellement caractérisé en ce qu'il comprend :
- un dôme 51, et
- un socle 52, comprenant une cavité, ou gorge 53, de forme convexe à la périphérie du dôme.

Le support 50 selon l'invention est avantageusement à axe de symétrie, en l'espèce par rapport à un axe Z-Z confondu avec l'axe optique, c'est à dire vertical. En l'espèce, l'axe de symétrie est un axe de symétrie de révolution.

Le dôme 51 du support 50 selon l'invention présente une forme convexe et reproduit la forme d'un oeil, sur lequel la membrane est posée pour la fabrication du dispositif selon l'invention.

De préférence, le dôme 51 du support 50 selon l'invention présente une forme sensiblement hémisphérique, dont le rayon est au plus égal au rayon équivalent de l'œil.

Dans un mode de réalisation le support 50 selon l'invention est caractérisé en ce que le dôme 51 peut être désolidarisé du socle 52, comme illustré sur la figure 2 où la position du dôme dans le socle 52 est représentée en pointillés. Dans ce mode de réalisation, le dôme 51 est amovible par rapport au socle 52.

Grâce à cette configuration, le dôme 51 et le socle 52 peuvent être réalisés dans des matériaux différents. Par exemple, le dôme 51 est en matériau plastique, et le socle 52 peut être en métal ou en alliage, en l'espèce en acier inoxydable. On peut ainsi mouler la membrane sur le dôme 51 en matériau plastique solidaire ou non du socle 52 du support 50, ce qui évite à la membrane de coller sur le dôme 51 lors du processus de lyophilisation par exemple.

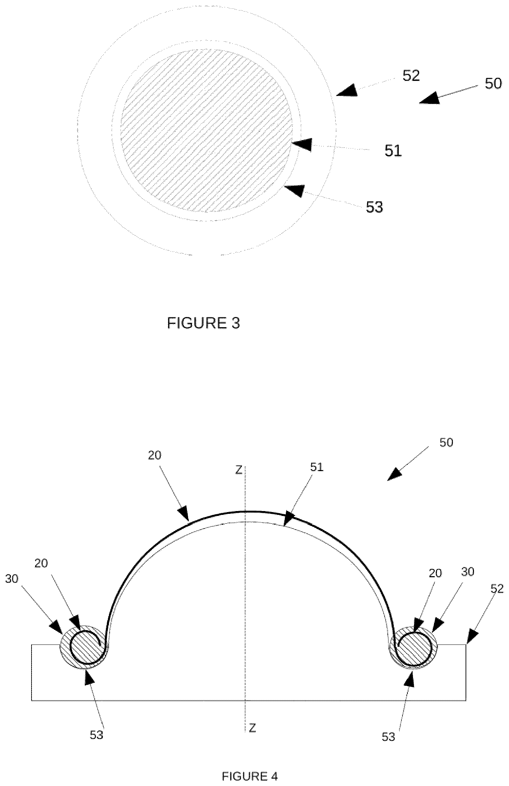

De préférence, le support 50 comprend une gorge 53 annulaire. En l'espèce, la gorge 53 est concentrique avec le dôme 51 et dans le prolongement de la base du dôme 51, comme illustré sur la figure 3. Ainsi, il n'y a pas d'angle entre la gorge 53 et le dôme 51, ce qui limite les risques de déchirer la membrane lors de la fabrication du dispositif selon l'invention.

La gorge 53 permet de mouler la structure annulaire 30, en particulier la gelée de Wharton, comme illustré sur la figure 4.

Avantageusement, la périphérie de la membrane peut ainsi être prise dans la masse de la structure annulaire 30.

Du fait que la périphérie de la membrane amniotique 20 soit prise dans la masse de la structure annulaire en matériau durcissable comme il est également visible sur les figures 4 et 5, aucune contrainte mécanique n'est appliquée à la membrane lors de l'assemblage du dispositif, ni lors de son utilisation.

La gorge 53 possède un diamètre choisi en fonction du diamètre de la structure annulaire désirée.

Dans un mode de réalisation, la gorge 53 est caractérisé en ce qu'elle possède un diamètre compris entre 1 et 3 cm.

Dans un mode de réalisation, la gorge 53 est caractérisé en ce qu'elle possède un diamètre compris entre 2 et 2.5 cm.

### Lentille imprégnée

Dans un mode de réalisation, le dispositif selon l'invention est caractérisé en ce qu'il est rempli dans sa partie interne par de la gelée de Wharton.

Dans un mode de réalisation, le dispositif selon l'invention est caractérisé en ce qu'il est rempli dans sa partie interne par de la gelée de Wharton comprenant un principe actif imprégné.

Par « imprégnation » on entend que le principe actif pénètre dans la substance et s'y répand, s'y diffuse.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des antifongiques, des anti-allergiques, des anesthésiques, ou des protéines immunoadhésives, des agents permettant d'éviter les sécheresses oculaires, seuls ou en combinaisons.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des antibiotiques. Quelques exemples d'antibiotiques utilisables sont donnés ci-après : tétracyclines (daunomycine, tétracycline, chloretetracycline, oxytétracycline, etc.), glycopeptides (vancomycine, etc.), aminoglycosides (gentamycine, etc.), aminosides (tobramycine, néomycine, etc.), fluoroquinolones (ciprofloxacine, moxifloxacin, etc.), quinolones (gatifloxacine, etc.), polypeptides (bacitracine, polymyxine, etc.), phénicolés (chloramphénicol, etc.), macrolides (erythromycine, etc.), sulfonamides (sulfacétamide, sulfaméthoxazole, sulfisoxazole, etc.), céphalosporines (céfradoxil, céfoxitine, etc.), et tout autre antibiotique.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des anti-inflammatoires stéroïdiens (AIS) et/ou non-stéroïdiens (AINS). Quelques exemples d'AIS sont donnés ci-après : triamcinolone, dexaméthasone, prednisolone, hydrocortisone, corticostérone, fluocinolone, prednisolone, méthylprednisolone, fluorométholone, betaméthasone, tétrahydrocortisol, riméxolone, etc. Quelques exemples d'AINS sont donnés ci-après : indométacine, népafénac, diclofénac, bromfénac, kétorolac, suprofène, etc.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des immunosuppresseurs, une liste non-limitative est donnée ci-après : déxaméthasone, bétaméthasone, etc.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des antiviraux, une liste non-limitative est donnée ci-après : ganciclovir, trifluorothymidine, aciclovir, DDI, AZT, foscarnet, vidarabine, trifluorouridine, idoxuridine, ribavirine, inhibiteurs de protéase, agent anti-cytomégalovirus, etc.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des antifongiques, une liste non-limitative est donnée ci-après : fluconazole, nitrofurazone, amphotéricine B, kétoconazole, etc.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des antiallergiques, une liste non-limitative est donnée ci-après : méthapyriline, chlorpheniramine, pyrilamine, prophenpyridamine, etc.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des anesthésiques, une liste non-limitative est donnée ci-après : lidocaïne, mépivacaïne, etc.

Dans un mode de réalisation, le dispositif selon l'invention est caractérisé en ce qu'il est rempli dans sa partie interne par de la gelée de Wharton comprenant un principe actif imprégné, ledit principe actif étant choisi dans le groupe constitué des agents permettant d'éviter les sécheresses oculaires, une liste non-limitative est donnée ci-après : azithromycine, ciclosporine, lubrifiants etc...

Dans un mode de réalisation, le dispositif selon l'invention rempli dans sa partie interne par de la gelée de Wharton comprenant un principe actif imprégné est caractérisé en ce qu'il est lyophilisé.

L'imprégnation de la gelée de wharton peut etre réalisée avant ou après lyophilisation.

La présente invention concerne également un procédé de fixation d'une membrane à un anneau comprenant les étapes de :
a) on dispose d'un support 50 ;
b) on prépare une première couche de gel 30 de forme annulaire ,
c) on dispose d'un anneau sur lequel est aposée ou enroulée une membrane amniotique 20 que l'on pose sur ladite première couche de façon à mettre en contact l'anneau et le gel;
d) on prépare une seconde couche de gel 30 de forme annulaire celle-ci étant appliquée sur la membrane et la première couche de façon à ce que la périphérie de la membrane et l'anneau soit entourés de gel ;
e) on procède à une étape de solidification du gel.

Dans un mode réalisation, le procédé de fixation d'une membrane à un anneau selon l'invention est caractérisé en ce qu'aux étapes b) et d) le gel 30 est de la gelée de Wharton.

Dans un mode réalisation, le procédé de fixation d'une membrane à un anneau selon l'invention est caractérisé en ce qu'à l'étape e) la solidification de la gelée de Wharton est réalisée par une lyophilisation.

Dans un mode réalisation, le procédé de fixation d'une membrane à un anneau selon l'invention est caractérisé en ce qu'à l'étape e) la solidification par lyophilisation de la gelée de Wharton est réalisée avec ajout de tréhalose.

### Exemples

### Exemple 1 : Exemples de modes de réalisation d'une lentille biologique selon l'invention.

### Fourniture d'une membrane amniotique viro-inactivée :

Un donneur proprement informé et consentant en accord avec les exigences de la Déclaration d'Helsinki offre en don le tissu placentaire issu d'un accouchement. Cet accouchement peut s'être déroulé par césarienne ou par voie basse. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le tissu placentaire est récupéré le plus tôt possible en salle d'accouchement suite à un accouchement, que ce soit par voie basse ou par césarienne. Il peut être avantageusement placé dans une boîte stérile puis congelé à une température de -20°C.

Ce tissu placentaire isolé peut être conservé à sec à une température de -80°C jusqu'à une durée de deux ans ou être directement traité.

Au laboratoire, en salle stérile, la procédure suivante est appliquée :
La membrane amniotique est isolée du placenta et est nettoyée.
La membrane amniotique subit une succession de bains assurant un traitement chimique de celle-ci. Ce traitement a pour objectif une désinfection de la membrane amniotique, et tout particulièrement sa viro-inactivation. Une agitation douce à environ 30 rpm du milieu liquide est appliquée lors de chaque bain afin d'assurer une pénétration homogène des solvants dans les tissus.

Dans un premier temps, la membrane amniotique est déposée dans un bain d'eau purifiée à température ambiante pendant environ 3 heures. Cette étape assure tant la décongélation du tissu physiologique qu'une première étape de lyse cellulaire par pression osmotique.

Puis, la membrane amniotique est transférée dans un bain décontaminant composé d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la membrane amniotique est transférée dans un bain composé de peroxyde d'hydrogène à 30% w/v à température ambiante pendant environ 15 minutes.

Puis la membrane amniotique est transférée dans un bain décontaminant composé de peroxyde d'hydrogène à 3% w/v à température ambiante pendant environ 1 heure.

L'action chimique appliquée à la membrane amniotique peut ensuite être neutralisée dans au moins un bain comportant, par exemple, de l'hydroxyde de sodium dilué autour d'un pH de 8,5. Le au moins un bain de neutralisation s'effectue à température ambiante pendant environ 15 minutes. On a trouvé des résultats intéressants à cette étape de neutralisation en utilisant deux bains à l'hydroxyde de sodium dilué à pH 8,5.

Afin d'assurer un rééquilibrage du pH et pour éliminer au mieux les résidus organiques se détachant du tissu d'intérêt, la membrane amniotique est transférée dans au moins un bain de tampon physiologique, choisi de façon appropriée, afin d'assurer son rééquilibrage physiologique. Le au moins un bain s'effectue à température ambiante pendant environ 15 minutes. On a trouvé des résultats intéressants à cette étape en utilisant deux bains successifs en solution de PBS.

Enfin, la membrane amniotique peut être transférée dans un dernier bain à l'eau purifiée, à température ambiante, pendant au moins 15 minutes et jusqu'à environ 1 heure.

A cette étape, on peut considérer que la membrane amniotique obtenue selon ce traitement chimique peut être définie comme une membrane amniotique en grande partie désinfectée notamment viro-inactivée, et décellularisée.

### Fourniture d'un broyat de gelée de Wharton :

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :

Un segment de 20 cm à 50 cm de longueur est isolé par section du cordon ombilical.

Le cordon ombilical est rincé et hydraté dans des bains d'eau purifiée successifs, sous agitation douce, pendant 4 heures.

Les vaisseaux sanguins du segment de cordon ombilical sont identifiés et séparés du reste du segment afin de conserver uniquement la gelée de Wharton et la membrane amniotique qui I 'entoure. La gelée de Wharton et la membrane amniotique sont utilisées dans la suite du procédé sous l'appellation générale de gelée de Wharton, car la quantité en poids de membrane est négligeable par rapport à la quantité en poids de gelée de Wharton.

La gelée de Wharton est congelée à sec à une température de -80°C.

Lors du traitement chimique, la gelée de Wharton est décongelée à l'air libre, à température ambiante, pendant une durée de 5 minutes. Cette étape de congélation, suivie d'une décongélation, assure une décellularisation importante du tissu biologique.

La gelée de Wharton subit une succession de bains assurant un traitement chimique de celle-ci. Ce traitement a pour objectif une désinfection de la gelée de Wharton, et tout particulièrement sa viro-inactivation. Une agitation linéaire douce du milieu liquide est appliquée lors de chaque bain afin d'assurer une pénétration homogène des solvants dans les tissus.

Dans un premier temps, la gelée de Wharton est déposée dans un bain d'eau purifiée à température ambiante pendant environ 3 heures. Cette étape assure tant la fin de la décongélation du tissu physiologique, qu'une étape de lyse cellulaire par pression osmotique.

Puis, la gelée de Wharton est transférée dans un bain décontaminant composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la gelée de Wharton est transférée dans un bain composé de peroxyde d'hydrogène à 30 % w/v à température ambiante pendant environ 15 minutes.

Puis, la gelée de Wharton est transférée dans un bain décontaminant composé de peroxyde d 'hydrogène à 3 % w/v à température ambiante pendant environ 1 heure. La gelée de Wharton obtenue est viro-inactivée. L'action chimique appliquée à la gelée de Wharton viro-inactivée est ensuite neutralisée, dans un bain comportant de l'hydroxyde de sodium dilué autour d'un pH de 8,5. Le traitement du bain de neutralisation s'effectue à température ambiante pendant environ 15 minutes.

Afin d'assurer un rééquilibrage du pH et pour éliminer au mieux les résidus organiques se détachant du tissu d'intérêt, la gelée de Wharton viro-inactivée est transférée dans un bain de tampon physiologique (PBS), afin d'assurer son rééquilibrage physiologique. Le bain s'effectue à température ambiante pendant environ 15 minutes.

Enfin, la gelée de Wharton est transférée dans deux bains successifs à l'eau purifiée, à température ambiante, pendant au moins 15 minutes et jusqu'à environ 1 heure.

A cette étape, on peut considérer que la gelée de Wharton obtenue selon ce traitement chimique est une gelée de Wharton en grande partie désinfectée, notamment viro-inactivée, et décellularisée.

Suite à cette partie relative aux traitements chimiques, la gelée de Wharton viro-inactivée subit une étape de broyage.

La gelée de Wharton est insérée dans un broyeur vibrant à billes Retsch MM400, équipé avec un bol en oxyde de zirconium de 35 mL. La gelée de Wharton occupe un espace d'environ 1/3 du bol. Une bille en oxyde de zirconium d'un diamètre de 20 mm est ajoutée dans le bol avec la gelée de Wharton. Un broyage de 1 minute est effectué à une fréquence de 3 Hz. La bille d'un diamètre de 20 mm est récupérée, et 9 billes en oxyde de zirconium d'un diamètre de 10 mm sont ajoutées dans le bol. Un deuxième broyage de 3 minutes est réalisé à une fréquence de 30 Hz. Un troisième broyage est effectué avec 60 billes de 5mm pendant 3 minutes à une fréquence de 30 Hz.

La substance obtenue est une substance liquide gélifiée homogène possédant la capacité de passer à travers une aiguille de diamètre 1,1mm ou 0,9 mm d'une seringue.

### Assemblage du dispositif selon l'invention

Premier mode de réalisation :

L'assemblage du dispositif est réalisé à l'aide d'un support 50 tel que décrit précédemment.

Une première couche de gelée de Wharton obtenue selon le mode opératoire précédent est injectée dans la gorge 53 du support 50, par exemple à l'aide d'une seringue.

Une membrane amniotique 20 obtenue selon le mode opératoire décrit précédemment est disposée sur le support 50 de manière à ce que celle-ci recouvre entièrement le dôme 51 du support 50 et que son contour externe repose sur la première couche de gelée de Wharton.

Une seconde couche de broyat de gelée de Wharton est injectée dans la gorge 53 du support 50 de façon à recouvrir le contour externe de la membrane amniotique 20 reposant sur la première couche de gelée de Wharton.

L'assemblage est ensuite lyophilisé.

À la suite du processus de lyophilisation, le contour de la membrane amniotique 20 se trouve « pris » dans la masse de gelée de Wharton qui s'est solidifiée.

Le dispositif de lentille 10 est ensuite conditionné et stérilisé afin d'être prêt à être utilisé.

### Second mode de réalisation :

L'assemblage du dispositif est réalisé à l'aide du support 50 tel que décrit précédemment.

Une première couche de gelée de Wharton obtenue selon le mode opératoire décrit précédemment est injectée dans la gorge 53 du support 50, par exemple à l'aide d'une seringue.

Un anneau de stabilisation 40 en polypropylène sur lequel est disposé une membrane amniotique 20 obtenue selon le mode opératoire précédent, les bords externes de la membrane se trouvant enroulés autour de l'anneau, est inséré dans la gorge 53 du support 50 recouvrant ainsi la première couche de gelée de Wharton.

Une seconde couche de gelée de Wharton est injectée dans la gorge 53 du support 50 de façon à ce que cette couche soit superposée à l'anneau de stabilisation 40, comme illustré sur la figure 5.

L'assemblage est ensuite lyophilisé.

À la suite du processus de lyophilisation, la membrane amniotique 20 et l'anneau de stabilisation 40 se trouvent « pris » dans la masse de gelée de Wharton qui s'est solidifiée.

Le dispositif de lentille 10 est ensuite conditionné et stérilisé afin d'être prêt à être utilisé.

La présente invention n'est pas limitée aux modes de réalisation précédemment décrits.

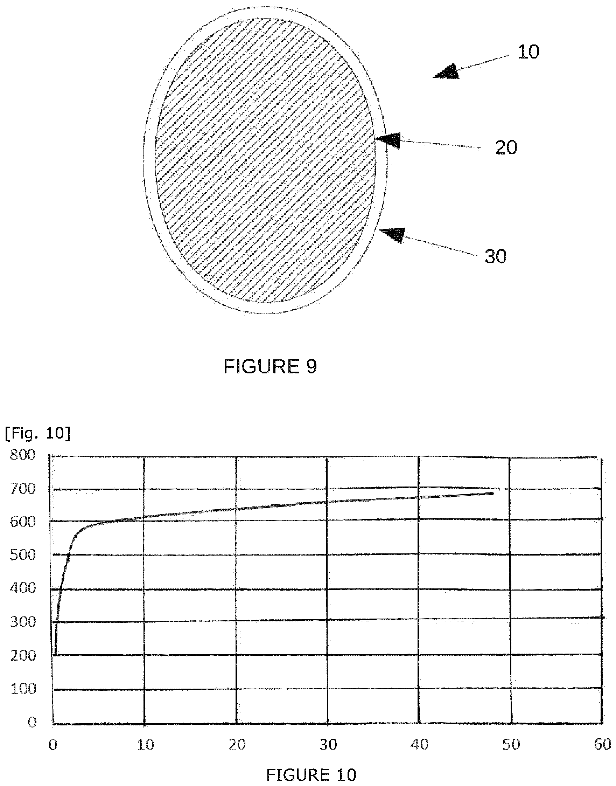

En particulier, on peut prévoir de prédécouper une membrane de forme ovale et de réaliser un dôme 51 également de forme ovale, ce qui permet de créer une lentille 10 de forme ovale, avec un fin anneau de stabilisation comme illustré sur la figure 10, qui peut avantageusement se positionner sur surface oculaire et se glisser sous la paupière.

### Exemple 2 : Dispositif selon l'invention rempli de gelée de Wharton imprégnée par un principe actif et titrage dudit principe actif.

Un dispositif selon l'invention est rempli avec de la gelée de Wharton et lyophilisé puis stérilisé.

200 µl d'une solution comprenant 2 mg de gentamycine est diluée dans 2 ml d'eau.

Les 2 ml de solution obtenue sont déposés sur la surface de la gelée de Wharton à l'aide d'une pipette.

La solution est absorbée par la gelée de Wharton et la quantité de gentamycine relarguée dans une solution d'eau saline renouvellée regulièrement après un pipetage complet, pendant 60 heures est évaluée par titration de chaque pipetage. Les résultats sont données dans le tableau suivant :

| Temps (H) | Activité totale (UI/ml) |
|---|---|
| 0,25 (15 minutes) | 206,06 |
| 0,5 (30 minutes) | 321,24 |
| 1,5 (90 minutes) | 471,66 |
| 4 | 584,81 |
| 24 | 644,09 |
| 48 | 682,56 |

La courbe de l'évolution de l'activité totale de gentamycine relarguée en UI/ml (axe des ordonnées) en fonction du temps en heures (axe des abscisses) est représentée sur le graphique en figure 10.

En conclusion, le dispositif est capable de diffuser une substance active de façon continue sur une longue période, en vu de traiter des infections complexes de la surface occulaire.

| | |
|---|---|
| 10 | lentille |
| 20 | membrane amniotique |
| 30 | structure annulaire |
| 40 | anneau de stabilisation |
| Z-Z | axe de symétrie |
| 50 | support |
| 51 | dôme |
| 52 | socle |
| 53 | gorge |

| | |
|---|---|
| Nomenclature | |

## Revendications

1. Dispositif, utilisable comme lentille, comprenant :
- une membrane amniotique (20) ;
- une structure annulaire (30) solidaire de ladite membrane et disposée à la périphérie de celle-ci,
- **caractérisé en ce que** ladite structure annulaire (30) est sous forme de gel durcissable choisi parmi le groupe des gels biocompatibles de polysaccharides, des gels de polymères de galactose, des gels de tissus biologiques.

2. Dispositif selon la revendication 1 **caractérisé en ce que** la structure annulaire (30) est en gelée de Wharton.

3. Dispositif selon l'une quelconque des revendications précédentes **caractérisé en ce que** la structure annulaire (30) possède un diamètre compris entre 1 et 3 cm.

4. Dispositif selon l'une quelconque des revendications précédentes, **caractérisé en ce que** la membrane amniotique (20) est viro-inactivée.

5. Dispositif selon la revendication 2, **caractérisé en ce que** la structure annulaire (30) en gelée de Wharton est viro-inactivée.

6. Dispositif selon la revendication 2, **caractérisé en ce que** la structure annulaire (30) en gelée de Wharton comprend au moins un principe actif choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des antifongiques, des anti-allergiques, des anesthésiques, ou des protéines immunoadhésives, des agents permettant d'éviter les sécheresses oculaires, seuls ou en combinaisons.

7. Dispositif selon l'une quelconque des revendications précédentes **caractérisé en ce que** la structure annulaire (30) comprend en outre un anneau de stabilisation (40).

8. Dispositif selon l'une quelconque des revendications précédentes **caractérisé en ce que** l'anneau de stabilisation (40) est de forme convexe.

9. Dispositif selon l'une quelconque des revendications précédentes **caractérisé en ce que** l'anneau de stabilisation (40) à une épaisseur comprise entre 0,1 mm et 3 mm.

10. Dispositif selon la revendication 8, **caractérisé en ce que** l'anneau de stabilisation (40) est un segment de vaisseau ombilical rempli de gelée de Wharton.

11. Dispositif selon l'une quelconque des revendications précédentes, **caractérisé en ce qu'**il est rempli dans sa partie interne par de la gelée de Wharton comprenant un principe actif imprégné.

12. Procédé de fabrication d'un dispositif utilisable comme lentille selon l'une quelconque des revendications 1 à 11 comprenant les étapes de :
a) on dispose d'un support (50) ;
b) on prépare une première couche de gel (30) de forme annulaire ;
c) on dispose une membrane amniotique (20) sur ladite première couche (30) de façon à mettre en contact la périphérie de ladite membrane et le gel ;
d) on prépare une seconde couche de gel (30) de forme annulaire celle-ci étant appliquée sur la membrane et la première couche de façon à ce que la périphérie de la membrane soit entourée de gel ;
e) on procède à une étape de solidification du gel et on obtient un dispositif utilisable comme lentille (10) biologique.

13. Procédé selon la revendication 12, **caractérisé en ce qu'**il comprend une étape de pose d'un anneau de stabilisation (40) entre l'étape c) et d).

14. Procédé selon la revendication 12 ou 13, **caractérisé en ce qu'**aux étapes b) et d) le gel (30) est de la gelée de Wharton.

15. Procédé selon la revendication 14, **caractérisé en ce qu'**à l'étape e) la solidification de la gelée de Wharton est réalisée par une lyophilisation.

16. Support permettant l'assemblage d'un dispositif utilisable comme lentille (10) selon l'une quelconque des revendications 1 à 11 **caractérisé en ce qu'**il comprend :
- un dôme (51)
- un socle (52), comprenant une cavité, ou gorge (53), de forme convexe à la périphérie du dôme.

17. Support selon la revendication 16, **caractérisé en ce que** le dôme (51) est désolidarisé du socle (52).

18. Procédé de fixation d'une membrane à un anneau comprenant les étapes de :
a) on dispose d'un support (50) ;
b) on prépare une première couche de gel (30) de forme annulaire ;
c) on dispose d'un anneau sur lequel est apposée ou enroulée une membrane amniotique (20) que l'on pose sur ladite première couche de façon à mettre en contact l'anneau et le gel;
d) on prépare une seconde couche de gel (30) de forme annulaire celle-ci étant appliquée sur la membrane et la première couche de façon à ce que la périphérie de la membrane et l'anneau soit entourés de gel ;
e) on procède à une étape de solidification du gel.

## Patentansprüche

1. Vorrichtung, verwendbar als Linse, umfassend:
- eine amniotische Membran (20);
- eine mit der Membran fest verbundene und an deren Rand angeordnete ringförmige Struktur (30),
**dadurch gekennzeichnet, dass** die ringförmige Struktur (30) in Form eines härtbaren Gels vorliegt, das ausgewählt ist aus der Gruppe aus biokompatiblen Gelen aus Polysacchariden, Gelen aus Galaktosepolymeren und Gelen aus biologischen Geweben.

2. Vorrichtung nach Anspruch 1, **dadurch gekennzeichnet, dass** die ringförmige Struktur (30) aus Wharton-Gelee ist.

3. Vorrichtung nach einem der vorangehenden Ansprüche, **dadurch gekennzeichnet, dass** die ringförmige Struktur (30) einen Durchmesser von 1 bis 3 cm aufweist.

4. Vorrichtung nach einem der vorangehenden Ansprüche, **dadurch gekennzeichnet, dass** die amniotische Membran (20) virus-inaktiviert ist.

5. Vorrichtung nach Anspruch 2, **dadurch gekennzeichnet, dass** die ringförmige Struktur (30) aus Wharton-Gelee virus-inaktiviert ist.

6. Vorrichtung nach Anspruch 2, **dadurch gekennzeichnet, dass** die ringförmige Struktur (30) aus Wharton-Gelee wenigstens einen Wirkstoff umfasst, der ausgewählt ist aus der Gruppe bestehend aus Antibiotika, Antiseptika, antiviralen Mitteln, monoklonalen Antikörpern, halbsynthetischen Metalloprotease-Inhibitoren, immunsupprimierenden Agenzien, entzündungshemmenden Mitteln, Antimykotika, Antiallergika, Anästhetika, oder immunadhäsiven Proteinen, Mitteln zur Vermeidung von trockenen Augen, einzeln oder als Kombinationen.

7. Vorrichtung nach einem der vorangehenden Ansprüche, **dadurch gekennzeichnet, dass** die ringförmige Struktur (30) außerdem einen Stabilisierungsring (40) umfasst.

8. Vorrichtung nach einem der vorangehenden Ansprüche, **dadurch gekennzeichnet, dass** der Stabilisierungsring (40) eine konvexe Form aufweist.

9. Vorrichtung nach einem der vorangehenden Ansprüche, **dadurch gekennzeichnet, dass** der Stabilisierungsring (40) eine Stärke zwischen 0,1 mm und 3 mm aufweist.

10. Vorrichtung nach Anspruch 8, **dadurch gekennzeichnet, dass** der Stabilisierungsring (40) ein Abschnitt eines mit Wharton-Gelee gefüllten Nabelschnurgefäßes ist.

11. Vorrichtung nach einem der vorangehenden Ansprüche, **dadurch gekennzeichnet, dass** sie in ihrem Inneren mit Wharton-Gelee gefüllt ist, das einen imprägnierten Wirkstoff umfasst.

12. Verfahren zum Herstellen einer als Linse geeigneten Vorrichtung nach einem der Ansprüche 1 bis 11, umfassend die folgenden Schritte:
a) Bereitstellen eines Trägers (50);
b) Herstellen einer ersten ringförmigen Gelschicht (30);
c) Anordnen einer amniotischen Membran (20) über der ersten Schicht (30), so dass der Rand der Membran und das Gel in Kontakt sind;
d) Herstellen einer zweiten ringförmigen Gelschicht (30), die auf die Membran und die erste Schicht so aufgetragen wird, dass der Rand der Membran von Gel umgeben ist; und
e) Verfestigen des Gels und Erhalten einer als biologische Linse (10) verwendbaren Vorrichtung.

13. Verfahren nach Anspruch 12, **dadurch gekennzeichnet, dass** es einen Schritt umfasst, bei dem ein Stabilisierungsring (40) zwischen den Schritten c) und d) angeordnet wird.

14. Verfahren nach einem der Ansprüche 12 oder 13, **dadurch gekennzeichnet, dass** in den Schritten b) und d) das Gel (30) ein Wharton-Gelee ist.

15. Verfahren nach Anspruch 14, **dadurch gekennzeichnet, dass** in Schritt e) das Verfestigen des Wharton-Gelees durch Lyophilisieren erfolgt.

16. Träger, der geeignet ist zum Zusammenfügen einer als Linse verwendbaren Vorrichtung (10) nach einem der Ansprüche 1 bis 11, **dadurch gekennzeichnet, dass** er umfasst:
- eine Kuppel (51) und
- einen eine Vertiefung oder Aushöhlung (53) umfassenden konvexen Sockel (52) am Rand der Kuppel.

17. Träger nach Anspruch 16, **dadurch gekennzeichnet, dass** die Kuppel (51) von dem Seckel (52) getrennt ist.

18. Verfahren zum Befestigen einer Membran an einem Ring umfassend die Schritte:
a) Bereitstellen eines Trägers (50);
b) Herstellen einer ersten ringförmigen Gelschicht (30);
c) Bereitstellen eines Ringes, auf dem eine amniotische Membran (20) aufgesetzt oder aufgerollt ist, der so auf der ersten Schicht aufgebracht wird, dass der Ring und das Gel in Kontakt sind;
d) Herstellen einer zweiten ringförmigen Gelschicht (30), wobei diese so auf die Membran und die erste Schicht aufgebracht wird, dass der Rand der Membran und der Ring von Gel umgeben sind; und
e) Verfestigen des Gels.

## Claims

1. A device that is suitable for use as a lens, comprising:
- an amniotic membrane (20);
- a ring structure (30) that is integral with said membrane and disposed at the periphery,
- **characterized in that** said ring structure (30) is in the form of a curable gel selected from the group of the biocompatible polysaccharide gels, galactose polymer gels, and biological tissue gels.

2. The device as set forth in claim 1, **characterized in that** the ring structure (30) is composed of Wharton's jelly.

3. The device as set forth in any one of the preceding claims, **characterized in that** the ring structure (30) has a diameter of between 1 and 3 cm.

4. The device as set forth in any one of the preceding claims, **characterized in that** the amniotic membrane (20) is viro-inactivated.

5. The device as set forth in claim 2, **characterized in that** the Wharton's jelly ring structure (30) is viro-inactivated.

6. The device as set forth in claim 2, **characterized in that** the ring structure (30) composed of Wharton's jelly comprises at least one active substance selected from the group consisting of antibiotics, antiseptics, antivirals, monoclonal antibodies, semi-synthetic metalloprotease inhibitors, immunosuppressive agents, anti-inflammatories, antifungals, antiallergics, anesthetics, or immunoadhesive proteins, or agents for preventing dry eyes, alone or in combination.

7. The device as set forth in any one of the preceding claims, **characterized in that** the ring structure (30) further comprises a stabilizing ring (40).

8. The device as set forth in any of the preceding claims, **characterized in that** the stabilizing ring (40) is convex in shape.

9. The device as set forth in any one of the preceding claims, **characterized in that** the stabilizing ring (40) has a thickness of between 0.1 mm and 3 mm.

10. The device as set forth in claim 8, **characterized in that** the stabilizing ring (40) is an umbilical vessel segment filled with Wharton's jelly.

11. The device as set forth in any one of the preceding claims, **characterized in that** it is filled on its interior with Wharton's jelly comprising an impregnated active substance.

12. A method for manufacturing a device that can be used as a lens as set forth in any one of claims 1 to 11, comprising the steps of:
a) providing a support (50);
b) preparing a first ring-shaped gel layer (30);
c) placing an amniotic membrane (20) on said first layer (30) so as to bring the periphery of said membrane and the gel into contact with one another;
d) preparing a second ring-shaped gel layer (30) and applying the same to the membrane and the first layer such that the periphery of the membrane is surrounded by gel;
e) carrying out a gel solidification step, thereby obtaining a device that can be used as a biological lens (10).

13. The method as set forth in claim 12, **characterized in that** it comprises, between steps c) and d), a step of placing a stabilizing ring (40).

14. The method as set forth in claim 12 or 13, **characterized in that**, in steps b) and d), the gel (30) is Wharton's jelly.

15. The method as set forth in claim 14, **characterized in that**, in step e), the solidification of the Wharton's jelly is performed by lyophilization.

16. A support for assembling a device that can be used as a lens (10) as set forth in any one of claims 1 to 11, **characterized in that** it comprises:
- a dome (51),
- a base (52) comprising a convex-shaped cavity, or groove (53), at the periphery of the dome.

17. The support as set forth in claim 16, **characterized in that** the dome (51) is detached from the base (52).

18. A method for securing a membrane to a ring, comprising the steps of:
a) providing a support (50);
b) preparing a first ring-shaped gel layer (30);
c) providing a ring on which an amniotic membrane (20) is apposed or wrapped and placing the same on said first layer such that the ring and the gel are brought into contact;
d) preparing a second ring-shaped gel layer (30) and applying the same to the membrane and the first layer such that the periphery of the membrane and the ring are surrounded by gel;
e) carrying out a gel solidification step.
